# FASCICULE DE BREVET EUROPEEN

(11) **EP 3 432 729 B1**
(45) Date de publication et mention de la délivrance du brevet: **13.08.2025**
(21) Numéro de dépôt: 17715241.0
(22) Date de dépôt: 17.03.2017
(51) Int. Cl.: A23K 40/30, A61K 9/51

(54) **NANOCAPSULES DE PRINCIPE ACTIF LIPOSOLUBLE, FABRICATION ET UTILISATIONS**
NANOKAPSELN MIT EINEM FETTLÖSLICHEN WIRKSTOFF, HERSTELLUNG UND VERWENDUNGEN
NANOCAPSULES COMPRISING A LIPOSOLUBLE ACTIVE INGREDIENT, PRODUCTION AND USES

(30) Priorité: 25.03.2016 FR 1652592
(43) Date de publication de la demande: 30.01.2019
(73) Titulaire: Adisseo France S.A.S., 92160 Antony (FR)
(72) Inventeur: PREVERAUD, Damien, 03310 Néris les Bains (FR); ROSILIO, Véronique, 75011 Paris (FR)
(74) Mandataire: Germain Maureau
(86) Numéro de dépôt international: PCT/FR2017/050622
(87) Numéro de publication internationale: WO 2017/162963

(56) Documents cités:
- EP-A1- 1 018 363
- EP-A1- 1 552 820
- WO-A2-2010/040194
- FR-A1- 2 803 203
- FR-A1- 2 924 943

## Description

L'invention concerne des nanocapsules de principes actifs liposolubles et leurs utilisations

L'invention est définie dans les revendications 1-12 attachées.

Les molécules, comme les vitamines, les acides gras, les huiles essentielles, sont très largement employées dans de nombreux domaines techniques tels que les industries pharmaceutique, cosmétique, agroalimentaire, et notamment dans le domaine de la nutrition animale. A titre d'exemple, les vitamines A et E sont couramment utilisées pour la préparation d'aliments favorisant la croissance et la santé d'animaux.

Leur nature hydrophobe et leur fragilité environnementale, notamment thermique et chimique, tant au cours de leur formulation et de leur stockage, que lors de leur utilisation, rendent nécessaire leur encapsulation.

La vitamine E, ou tocophérol (TOL en abrégé) existant majoritairement sous la forme d-α-tocophérol (αTOL), est, à l'état natif, un liquide huileux, lipophile, miscible en toutes proportions dans toute phase hydrophobe ou lipidique. Elle est extrêmement instable, et aisément oxydable, et, à l'état oxydé, elle perd l'essentiel de son activité biologique. Sa biodisponibilité chez l'animal n'excède pas 50% quand elle est administrée par voie orale, car, rapidement oxydée, elle est majoritairement absorbée dans cette forme oxydée, inactive. Aussi, quand elle est administrée par voie orale, la vitamine E l'est sous la forme d'un dérivé plus stable, généralement choisi parmi les esters, par exemple l'acétate, et les sels de vitamine E.

La vitamine A existe sous plusieurs formes, notamment à l'état d'ester, et c'est sous l'une de ses formes les plus stables, l'acétate de rétinyle, qu'elle est le plus souvent consommée par les animaux d'élevage (volaille, porcs et bovins). Elle reste toutefois sensible à l'oxydation, à la température, à la lumière, aux acides. En application pharmaceutique ou en nutrition animale, elle est ainsi très rapidement dégradée dès qu'elle entre en contact avec les premières conditions sévères, notamment acides, du système digestif, ce qui n'en fait pas une forme biodisponible de la vitamine A.

Afin de préserver au mieux ces principes actifs sensibles, il est connu depuis longtemps qu'on peut les protéger par enrobage ou encapsulation. Diverses voies d'encapsulation des vitamines notamment A et E ont été développées et largement utilisées, comme celle impliquant des protéines.

On est toutefois toujours à la recherche d'une formulation d'un principe actif liposoluble qui serait hautement biodisponible.

Les auteurs ont recherché une nouvelle formulation de tels principes actifs qui soit capable d'augmenter leur absorption notamment leur absorption intestinale.

La plupart de ces principes actifs étant généralement utilisés dans leur forme protégée, il était en outre essentiel de développer une formulation qui permette que lesdits principes actifs soient absorbés dans leur forme libre, active, ce qui signifie que l'hydrolyse de la forme protégée et l'absorption doivent se produire quasiment simultanément.

Selon les documents suivants, on connait diverses préparations de principes actifs sous forme de particules.

EP1552820A1 divulgue une dispersion aqueuse de nanocapsules, lesdites nanocapsules comprenant une fraction huileuse pouvant consister en vitamine E, F ou K, leurs esters et leurs mélanges, destinées à une utilisation en cosmétique et pharmacie. Ces nanocapsules sont constituées d'un cœur de ladite huile comprenant aussi un ou plusieurs tensioactifs non ioniques facilitant la préparation et d'une coquille polymère.

FR2924943A1 décrit aussi une dispersion aqueuse de nanocapsules, lesdites nanocapsules comprenant une fraction huileuse dans laquelle est dissous un dérivé de la vitamine D. Plus précisément, ces nanocapsules sont constituées d'une fraction huileuse de principe actif dissous dans l'huile, ladite fraction étant entourée d'une enveloppe de polymère hydrophobe, cette dernière étant elle-même entourée d'un agent d'enrobage sous forme d'une phase lamellaire externe et constitué par un tensioactif hydrophobe, ladite phase lamella qui peut elle-même être enrobée par un agent gélifiant.

EP1018363A1 décrit une nanoémulsion H/E consistant en des nanoglobules d'huile dispersées dans une phase continue aqueuse et contenant un tensioactif choisi parmi les copolymères blocs d'oxyde d'éthylène et d'oxyde de propylène, notamment destinée à une utilisation cosmétique.

FR2803203A1 concerne une formulation de fénofibrate destinée à une administration par voie orale et capable de former une microémulsion H/E au contact d'un milieu aqueux, tel qu'un milieu biologique, et comprenant une phase lipophile qui contient une huile, du fénofibrate, de l'acétate de vitamine E permettant de stabiliser le fénofibrate dans cette phase et un tensioactif lipophile et un co-tensioactif hydrophile. Des capsules molles formées à partir de cette phase sont aussi décrites.

WO2010/070194A2 décrit des nanoparticules qui peuvent être des nanocapsules comprenant un cœur d'un filtre UV et d'une huile et un revêtement polymère, destinée à une utilisation en cosmétique.

Les auteurs ont d'abord découvert que de tels principes actifs pouvaient être formulés en nanocapsules à haute teneur en dits principes actifs, et cela grâce à un procédé propre au sens où il n'a recours à aucun solvant organique. Puis les auteurs ont mis au point des nanocapsules capables de libérer, de manière efficiente, la forme active du principe actif, à savoir, dans une forme satisfaisant l'ensemble des impératifs ci-dessus.

Ainsi l'invention concerne une formulation de principe(s) actif(s) liposoluble(s) en teneur élevée, sous forme de nanocapsules, laquelle présente une biodisponibilité supérieure aux formulations du marché actuel.

La mise en œuvre d'un procédé de fabrication industrialisable et respectueux de l'environnement, pour obtenir de telles nanocapsules, est décrite et non revendiquée. De plus, le procédé mis au point par les auteurs conduit à des nanocapsules possédant une humidité résiduelle basse, de préférence inférieure à 8%, ce qui leur confère une stabilité dans le temps, quelles que soient les conditions de stockage.

Selon le domaine d'application du principe actif, les nanocapsules de l'invention peuvent être mises sous une forme en vue d'une manipulation plus facile, par exemple sous forme de microparticules, notamment par adsorption desdites nanocapsules sur un support. Dans la suite de la description, le terme « particule » sera réservé à toute présentation desdites nanocapsules, et à titre d'exemple, de telles particules sont des microparticules comprenant des nanocapsules de l'invention. Si le principe actif est destiné à la nutrition animale, il est ainsi particulièrement avantageux que les nanocapsules soient formulées en particules sèches présentant une excellente mixabilité pour leur incorporation dans un prémix. Dans cette indication, de telles particules sont des microparticules d'une taille moyenne inférieure à 300 µm.

Les différents objets de l'invention vont maintenant être exposés en détail.

L'invention est ci-après plus particulièrement décrite en référence à la vitamine E, mais bien entendu, son cadre n'y est pas restreint, et elle s'applique à toute substance active liposoluble et tout mélange de telles substances.

Comme dit précédemment, l'invention a pour objet des nanocapsules comprenant au moins un principe actif liposoluble, en une concentration élevée, qui sont stables et qui peuvent être hautement biodisponibles. Les nanocapsules de l'invention peuvent se présenter sous forme d'une suspension colloïdale, ou sous forme sèche, après séchage de cette suspension.

Qu'elles soient sous forme d'une suspension colloïdale, ou sous forme sèche, lesdites nanocapsules comprennent une fraction huileuse consistant en un principe actif liposoluble et un tensioactif ionique,
un tensioactif non ionique,
et un polymère hydrophile, cationique ou anionique, entourant ladite fraction huileuse,
lesdites nanocapsules répondant aux caractéristiques suivantes,
la charge du tensioactif ionique et celle du polymère hydrophile sont opposées, ledit tensioactif ionique étant choisi parmi les phosphatidylcholines et le bromure d'hexadécyl triméthylammonium,
le tensioactif non ionique est choisi parmi les copolymères à blocs polyoxyéthylène-polyoxypropylène, les mélange de copolymères à blocs polyoxyéthylène (EO)-polyoxypropylène (PO), le Tween 80, les esters d'acides gras et de saccharose, et tout mélange de ceux-ci, et
ledit polymère est choisi parmi le chitosane, l'alginate, la pectine, l'amidon, la cellulose, la caséine et leurs combinaisons.

Les auteurs ont découvert de manière inattendue que pour rendre la vitamine E biodisponible, ou à tout le moins augmenter sa biodisponibilité, les nanocapsules devaient en outre comprendre au moins un tensioactif non ionique.

Comme déjà évoqué, les nanocapsules de l'invention permettent de véhiculer tout principe actif liposoluble. Ainsi, celui-ci peut être choisi parmi :
les vitamines liposolubles telles que les vitamines A, D, E, K, leurs dérivés, notamment esters par exemple acétate, propionate ou succinate, ainsi que leurs métabolites comme le rétinal, l'acide rétinoïque, le 25-hydroxycholecalciférol, le 1,25 dihydroxycholecalciférol ;
les caroténoïdes ;
les huiles essentielles telles que des huiles essentielles de thym, d'origan, de romarin, d'ail, de camélia, de moutarde, de gingembre, de curcuma, de raisin, d'agrumes, de sainfoin, de yucca, d'armoise, de cannelle, de menthe, de clou de girofle, de baies, de cumin et d'Echinacea ;
les acides gras, saturés, mono-insaturés et poly-insaturés ;
les huiles grasses.

Si le principe actif est liquide ou susceptible de l'être par chauffage, il peut constituer à lui-seul la phase huileuse dans laquelle le tensioactif ionique sera présent.

S'il n'est pas à l'état liquide à la température de fabrication des nanocapsules, il peut être préalablement solubilisé dans une huile, généralement inerte, qui servira de support. A titre d'exemple, cette huile peut être la trioléine.

Un intérêt des nanocapsules de l'invention réside dans leur teneur en principe actif qui peut varier d'un minimum par exemple de 5% en masse par rapport à la masse sèche des nanocapsules (m/m), à plus de 50%, voire même au moins 90%. Cette teneur sera déterminée en fonction de la destination des nanocapsules, elle est généralement d'au moins 5% en masse par rapport à la masse sèche des nanocapsules (m/m), de préférence d'au moins 25%, et mieux encore d'au moins 50%, voire d'au moins 70%, même d'au moins 90%.

Ledit tensioactif ionique est de préférence choisi parmi ceux dont le poids moléculaire est d'au plus 1500 g/mol, voire d'au plus 1000 g/mol. Au-dessus de 1500 g/mol, les nanocapsules sont difficilement formées. Parmi les phosphatidylcholines, on peut retenir la lécithine d'œuf ou la lécithine de soja. Comme dit précédemment, le tensioactif ionique, chargé positivement ou négativement, est sélectionné pour être de charge opposée à celle du polymère.

Dans le cadre de la définition donnée d'un tensioactif non ionique, les tensioactifs préférés sont choisis parmi les copolymères de formule EOₓ-PO_{y}-EOₓ dans laquelle x varie de 75 à 85 et y varie de 25 à 35, les copolymères de formule EOₓ-PO_{y}-EOₓ dans laquelle x varie de 55 à 65 et y varie de 35 à 45 et les copolymères de formule EOₓ-PO_{y}-EOₓ dans laquelle x varie de 112 à 123 et y varie de 40 à 50, ainsi que les esters d'acides gras et de saccharose commercialisés sous les marques SISTERNA^{®} SP70 et PS750.

Le ou les polymères permettant d'obtenir des nanocapsules selon l'invention sont choisis parmi des polymères cationiques ou anioniques, la charge du polymère étant opposée à celle du tensioactif ionique.. Ainsi, les combinaisons polymère-tensioactif ionique préférées sont celles constituées par le chitosane et la lécithine d'œuf, et l'alginate et le CTAB.

Selon une variante préférée de l'invention, la teneur du tensioactif non ionique tel que défini ci-dessus, dans la suspension, est d'au moins 15% en masse par rapport à la masse sèche des nanocapsules (m/m).

Un autre des objets de l'invention consiste en des nanocapsules issues du séchage de la suspension colloïdale décrite ci-dessus. Ces nanocapsules comprennent donc
une fraction huileuse consistant en un principe actif liposoluble et un tensioactif ionique,
un tensioactif non ionique,
et un polymère hydrophile, cationique ou anionique, entourant ladite fraction huileuse,
lesdites nanocapsules répondant aux caractéristiques suivantes,
la charge du tensioactif ionique et celle du polymère hydrophile sont opposées, ledit tensioactif ionique étant choisi parmi les phosphatidylcholines et le bromure d'hexadécyl triméthylammonium,
le tensioactif non ionique est choisi parmi les copolymères à blocs polyoxyéthylène-polyoxypropylène, les mélange de copolymères à blocs polyoxyéthylène (EO)-polyoxypropylène (PO), le Tween 80, les esters d'acides gras et de saccharose, et tout mélange de ceux-ci, et
ledit polymère est choisi parmi le chitosane, l'alginate, la pectine, l'amidon, la cellulose, la caséine et leurs combinaisons,
lesdites nanocapsules étant susceptibles d'être obtenues par séchage d'une suspension colloïdale de l'invention. Le séchage est avantageusement réalisé en présence de lactose, les nanocapsules de l'invention étant adsorbées sur le lactose.

L'invention apporte aussi des particules comprenant des nanocapsules telles que décrites précédemment, lesdites nanocapsules étant adsorbées sur un support. Ce support peut être choisi parmi tout support inerte, comme par exemple le lactose. Dans une variante préférée, ces particules sont des microparticules comprenant des nanocapsules de l'invention adsorbées sur du lactose.

Un procédé de fabrication de nanocapsules de l'invention, qu'elles soient dans une suspension colloïdale ci-dessus ou à l'état sec après traitement d'une telle suspension est décrit ci-après.

Ainsi, un procédé de fabrication d'une suspension colloïdale de nanocapsules comprend les étapes suivantes :
- On dispose d'une première phase comprenant au moins une fraction huileuse comprenant ou consistant en au moins un principe actif liposoluble et un tensioactif ionique d'une part, et d'une seconde phase aqueuse comprenant au moins un polymère et éventuellement un tensioactif non ionique d'autre part, la concentration molaire dudit tensioactif ionique et celle du tensioactif non ionique, le cas échéant, étant supérieures ou égales à 100 fois la concentration micellaire critique (CMC) dudit ou desdits tensioactifs ioniques et dudit ou desdits tensioactifs non ioniques, respectivement ; le ou les principes actifs, tensioactifs ioniques, tensioactifs non ioniques et polymères répondant aux définitions données précédemment;
- On forme une émulsion grossière qu'on homogénéise ensuite sous haute pression pour former la suspension colloïdale de nanocapsules.

La détermination de la CMC peut être effectuée par toute technique bien connue de l'homme du métier, par exemple par des mesures de tension superficielle par un tensiomètre à lame ou anneau.

Si le ou les principes actifs ne sont pas liquides à la température ambiante ou trop visqueux, les phases huileuse et aqueuse sont portées à une température variant de 60 à 70°C, permettant la fusion du ou des principes actifs.

L'émulsion dite grossière est obtenue par simple agitation des phases aqueuse et huileuse. Son homogénéisation est ensuite réalisée sous haute pression, par exemple pendant au moins 6 minutes, à une pression de préférence au moins égale à 600 bar.

Afin d'obtenir des nanocapsules sèches selon l'invention à partir d'une suspension colloïdale de nanocapsules telle que décrite ci-dessus, on sèche par atomisation en présence de lactose lesdites nanocapsules. Ce procédé conduit à des particules qui sont non collantes et qui peuvent être conservées à température ambiante.

L'invention concerne aussi les utilisations de telles nanocapsules. Elles présentent un grand intérêt en nutrition animale, notamment pour les animaux monogastriques. Dans cette indication, elles sont utilisées sous forme de particules, et notamment sous forme de microparticules, telles que décrites précédemment.

L'invention est illustrée et ses avantages mis en lumière dans les exemples suivants exposant la fabrication de nanocapsules d'acétate d'alpha-tocophérol (αTAC) et leurs performances en nutrition animale dans des essais *in vitro* et *in vivo.*
La figure 1 représente le taux de bioaccessibilité *in vitro* du TAC de différentes formulations de TAC.
La figure 2 représente le taux d'hydrolyse *in vitro* de TAC en TOL de différentes formulations de TAC.
La figure 3 représente la concentration plasmatique en αTOL (en µM) chez le rat après administration par gavage de différentes formulations de TAC.
Les figures 4 et 5 représentent la concentration plasmatique en αTOL (en µg/ml) chez le coq après administration par gavage de différentes formulations de TAC.
La figure 6 représente la concentration plasmatique en αTOL (en µg/ml) chez le poulet après administration dans l'aliment de différentes formulations de TAC.

Dans les exemples suivants, divers paramètres sont analysés et en particulier la biodisponibilité en vitamine E.

La biodisponibilité d'un principe actif liposoluble, tel que la vitamine E, ou d'un dérivé de la vitamine E est représentée par la concentration de vitamine E libérée dans le sang, par rapport à la concentration de vitamine E présente dans la ration de l'animal, ou par rapport à la concentration exprimée en équivalent de vitamine E du dérivé de vitamine E introduit dans la ration de l'animal lorsque l'on administre un dérivé de la vitamine. Cette représentation de la biodisponibilité de la vitamine E prend donc en compte l'absorption de la vitamine E ou du dérivé de la vitamine E dans l'intestin au cours du transit digestif.

### Exemple 1 : Fabrication de nanocapsules selon l'invention et hors invention

### Formulation

Les nanocapsules préparées dans cet exemple sont identifiées par les références C24 (invention), A37 (invention) et C40 (hors invention).

Elles sont obtenues à partir d'une suspension colloïdale comprenant au moins :
- du TAC,
- un tensioactif ionique choisi parmi la lécithine d'œuf (Lipoid E80) et le bromure d'hexadécyl triméthylammonium (CTAB),
- pour les nanocapsules C24 et A37, un tensioactif non ionique le Lutrol^{®}-F68,
- au moins un polymère hydrophile ionique choisi parmi le chitosane (cationique) et l'alginate de sodium (anionique),
la dite suspension colloïdale étant ensuite séchée en présence de lactose.

La formulation de ces nanoparticules figure dans le tableau 1 suivant, la teneur des ingrédients étant exprimée en % (m/m de matières sèches) :

**Tableau 1**

| Particules | | C24 | A37 | C40 |
|---|---|---|---|---|
| Principe actif | TAC | 24 | 37 | 39 |
| Tensioactif ionique | Lipoid E80 | 12 | - | 10 |
| | CTAB | - | 4,5 | - |
| Tensioactif non ionique | Lutrol^{®}-F68 | 15 | 23 | - |
| Polymère | Chitosane | 13 | - | 10 |
| | Alginate de sodium | - | 8,1 | |
| Support | Lactose | 36 | 27,4 | 41 |

### Fabrication

Le procédé de fabrication des nanoparticules compte les 3 étapes suivantes :
préparation de la nanoémulsion,
préparation de la suspension colloïdale de nanocapsules, et
séchage des nanocapsules.

### Protocole de fabrication des nanocapsules C24 et A37 :

### Préparation de la nanoémulsion :

Pour les nanocapsules C24 :
- on disperse le tensioactif ionique, Lipoid E80, dans le TAC, sous agitation à la turbine, et on porte la dispersion à 65°C, pour obtenir une phase huileuse,
- on dissout le tensioactif non ionique (Lutrol^{®} F68) dans l'eau, et on porte la solution à 65°C, pour obtenir une phase aqueuse,

Pour les nanocapsules A37 :
- on porte le TAC qui constitue la phase huileuse, à 65°C, et on y disperse le tensioactif ionique, CTAB,
- le tensioactif non ionique (Lutrol^{®} F68) est dissous dans l'eau et on porte la solution à 65°C, pour obtenir une phase aqueuse,
puis pour les nanocapsules C24 et A37 :
- on ajoute la phase aqueuse à la phase huileuse sous agitation et on forme une émulsion primaire ou grossière à l'aide d'une turbine Reyneri 600 tours/min, à 65°C, pendant 15 minutes,
- on transfère l'émulsion dans l'homogénéisateur haute pression et on l'homogénéise à la pression de 600 bars pendant 6 minutes à 65°C, pour obtenir la nanoémulsion.

Préparation de la suspension colloïdale des nanocapsules :
- on dilue au dixième la nanoémulsion obtenue ci-dessus par une solution de Lutrol^{®} F68,
- on ajoute la solution acétique de chitosane à 0,05 g/L pour les nanocapsules C24 ou la solution d'alginate de sodium à 1,8 g/L pour les nanocapsules A37, sous la turbine et on agite pendant 2h à température ambiante, pour obtenir une suspension colloïdale de nanocapsules, selon l'invention.

Séchage des nanocapsules :
- on sèche les nanocapsules par atomisation sur lactose ; les paramètres sont un débit de pompe de 15%, une température d'entrée de 150°C, un débit de 7 mL/min et un flux d'air comprimé de 500 L/h.

### Protocole de fabrication des nanocapsules C40 :

Les nanocapsules C40 sont fabriquées selon le procédé décrit ci-dessus pour les nanocapsules C24, à l'exception du fait qu'aucun tensioactif non ionique n'est ajouté.

### Caractérisation des nanocapsules :

Les nanocapsules sont caractérisées par leur taille indiquée dans le tableau 2 suivant, à deux étapes du procédé de fabrication, la première à la formation de la nanoémulsion, avant l'ajout du polymère et la seconde à l'issue du procédé avant séchage des nanocapsules :

**Tableau 2**

| Nanoparticules | C24 | A37 | C40 |
|---|---|---|---|
| Taille des nanogouttelettes, avant ajout du polymère (nm) | 219 | 236 | 123 |
| Taille des nanocapsules avant séchage (nm) | 355 | 342 | 163 |

Les exemples suivants illustrent l'intérêt des formulations de TAC selon l'invention par l'évaluation de la biodisponibilité du TAC.

La biodisponibilité du TAC d'une formulation correspond à la proportion de TOL absorbée par la muqueuse intestinale qui servira pour le métabolisme cellulaire et les fonctions organiques. Cette biodisponibilité est la combinaison de différents facteurs, et notamment la bioaccessibilité du TAC, c'est-à-dire la proportion de vitamine E présente dans une formulation (sous forme de TAC) qui se retrouve solubilisée dans les micelles mixtes, et l'hydrolyse du TAC en TOL par la carboxy ester hydrolase (CEH) sécrétée dans le système digestif.

### Exemple 2 : Bioaccessibilité du TAC dans des formulations de TAC évaluée dans des essais in vitro

Ce test est décrit par Desmarchelier et al., 2013. Mol. Nutr. Food Res. 2013, 57, 1237-1245.

Dans ces essais *in vitro,* on prépare des micelles mixtes contenant différentes formulations de TAC de l'exemple 1 qui permettent de reproduire les conditions de la digestion en imitant les micelles impliquées dans l'intestin.

La bioaccessibilité de la vitamine E est calculée après digestion *in vitro* de l'aliment contenant les différentes formulations. Elle est déterminée par le rapport entre la vitamine E dosée par HPLC se retrouvant dans la phase micellaire, et la vitamine E dosée par HPLC présente dans le digestat obtenu en fin de phase duodénale.

Les essais sont réalisés sur trois types de formulation :
- les nanocapsules C24 et A37 de l'exemple 1 : les nanocapsules A37 sont testées sous deux formes : une forme poudre avec mise sur support sur lactose (A37); et
- un produit identifié par la référence Promix, consistant en une huile de vitamine E adsorbée sur silice (Promix et Promix (2) sont deux répétitions du même produit)

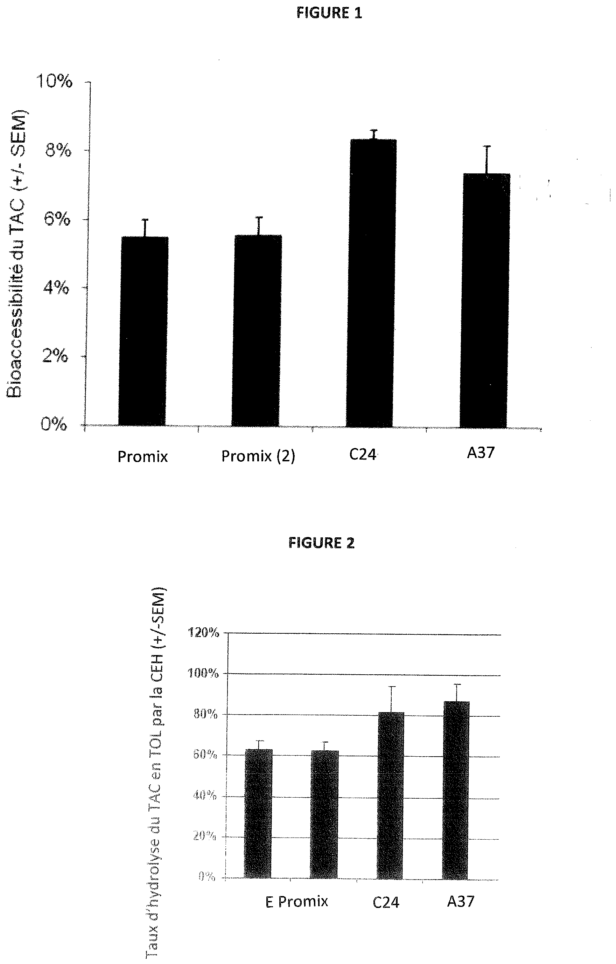

Les résultats sont représentés à la Figure 1.

On observe que les nanocapsules de TAC (C24 et A37) permettent d'augmenter la bioaccessibilité de la vitamine E par rapport au produit Promix, c'est-à-dire, la quantité de vitamine E contenue dans la matrice alimentaire qui est solubilisée dans les micelles mixtes à l'issue d'une digestion *in vitro.*

### Exemple 3 : Hydrolyse du TAC en TOL par la CEH dans des essais in vitro

Le protocole d'hydrolyse de la vitamine E-acétate (TAC) par la CEH est décrit par Desmarchelier et al., 2013. Mol. Nutr. Food Res. 2013, 57, 1237-1245.

Brièvement, 500 µl de micelles mixtes contenant le TAC sont incubés 30 min à 37°C. De la CEH est ensuite ajoutée à une concentration de 10 U/mL pendant 30 min. L'apparition de tocophérol libre (TOL) est ensuite mesurée par HPLC.

Les essais sont réalisés sur trois types de formulation :
- les nanocapsules C24 et A37 de l'exemple 1 ; et
- un produit identifié par la référence E Promix, consistant en une huile de vitamine E.

Les résultats sont représentés à la Figure 2.

Les nanocapsules de TAC (C24 et A37) permettent d'augmenter la conversion de TAC en TOL par rapport au produit E Promix, ce qui résulte en une plus grande quantité de vitamine E disponible pour l'absorption.

### Exemple 4 : Biodisponibilité du TAC dans des essais in vivo chez le rat

### Protocole

Les essais sont réalisés sur des rats Wistar mâle âgés de 6 semaines nourris pendant 2 semaines avec un aliment carencé en tocophérol.

Les rats ont été mis à jeun la nuit précédant le gavage.

Les rats (n = 10) ont été gavés pendant 5 jours consécutifs avec 5 mg de différentes solutions de TAC dans de l'eau :
- Microvit^{®} E Promix 50 (acétate de vitamine E adsorbé sur silice),
- Nanocapsules sèches C24 de l'exemple 1, et
- Nanocapsules sèches A37 de l'exemple 1.

Trois heures après le dernier gavage, les rats ont été anesthésiés, du sang a été prélevé par ponction intracardiaque et, après centrifugation, le plasma a été isolé. Après une double extraction hexanique, la concentration plasmatique en alpha-tocophérol (αTOL) a été mesurée par HPLC.

### Résultats

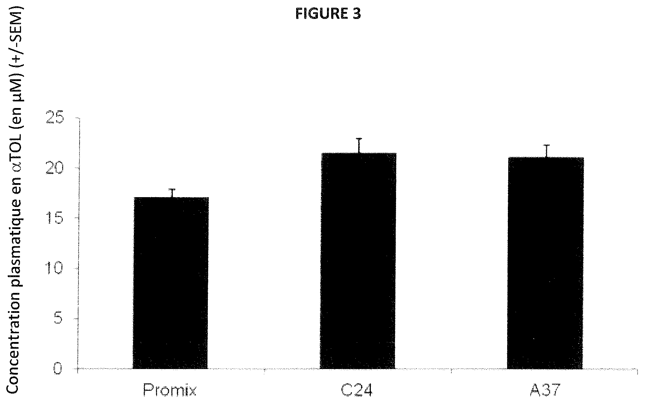

Les résultats sont reportés dans la figure 3.

Le gavage avec les formulations C24 et A37 a conduit à des concentrations plasmatiques en αTOL, respectivement 26% et 24%, significativement plus élevées (P<0.001) que le gavage avec le Microvit^{®} E Promix 50.

### Exemple 5: Biodisponibilité du TAC dans des essais in vivo chez le coq

### Protocole

Le schéma expérimental est décrit en détail dans Prévéraud et al. 2015, British Poultry Science, 56 :1 ; 94-102.

Brièvement, deux salles de 60 coqs ISA Brown sont placées dans des cages individuelles. Une semaine avant l'attribution des traitements, les coqs sont alimentés avec un aliment dépourvu en vitamine E.

Les coqs (n=10 coqs par traitement) ont été gavés avec différentes solutions de TAC dans l'eau.

| | |
|---|---|
| Salle 1 : | Nanocapsules sèches C24 de l'exemple 1, TOL et TAC, sous forme d'huile, à titre de témoin. |
| Salle 2 : | Nanocapsules sèches C40 de l'exemple 1, TOL et TAC, sous forme d'huile, à titre de témoin. |

Après l'administration par gavage des produits vitamine E mis dans une gélule, des prélèvements de sang ont été fait à 0, 6, 12, 24, 48 et 96 h post gavage. Après centrifugation, le plasma est décanté et le tocophérol libre est dosé par HPLC.

### Résultats

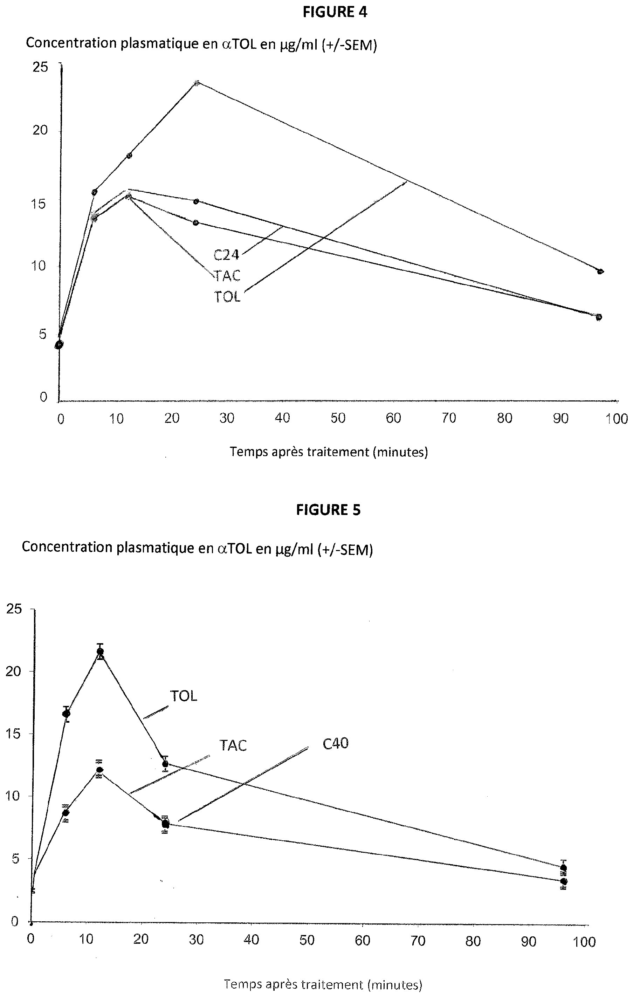

Les résultats sont illustrés aux figures 4 et 5 et reportés pour les temps à 24h et à 96h dans les tableaux 3 et 4 suivants, dans lesquels la diffusion de la vitamine E dans le sang est exprimée par l'aire sous la courbe (AUC) et le pourcentage de diffusion par rapport au TAC.

**Tableau 3**

| Vit E (Salle 1) | 24h | | 96h | |
|---|---|---|---|---|
| | AUC µg/ml/h | %/TAC | AUC µg/ml/h | %/TAC |
| TAC | 206 | - | 597 | - |
| TOL | 307 | +49% | 1193 | +100% |
| C24 | 228 | +11% | 690 | +16% |

Le gavage avec la formulation C24 a conduit à des concentrations plasmatiques en αTOL significativement plus élevées que le gavage avec le TAC non formulé.

**Tableau 4**

| Vit E (Salle 2) | 24h | | 96h | |
|---|---|---|---|---|
| | AUC µg/ml/h | %/TAC | AUC µg/ml/h | %/TAC |
| TAC | 142 | - | 340 | - |
| TOL | 309 | +118% | 320 | +124% |
| C40 | 146 | +3% | 719 | +6% |

Le gavage avec la formulation C40 a conduit à des concentrations plasmatiques en αTOL non significativement différentes de celles du témoin TAC non formulé, et préparée en l'absence d'un tensioactif non ionique, elle ne présente donc pas de potentiel de biodisponibilité.

### Exemple 6: Biodisponibilité du TAC dans des essais in vivo chez le poulet

### Protocole

Des poulets de 1 jour nourris pendant 7 jours avec un aliment carencé en tocophérol sont mis à l'étude ; la durée totale de la phase expérimentale est fixée à 15 jours au cours de laquelle ils reçoivent différents traitements de vitamine E mélangée à l'aliment granulé (n=18 par traitement). Au préalable, les poulets ont été placés par groupe de 6 par cage. A l'âge de 21 jours, les animaux sont euthanasiés et des prélèvements de foie sont réalisés. Après extraction, la vitamine E est dosée dans ce tissu.

Les poulets ont été alimentés par les formulations suivantes:
- Microvit^{®} E Promix 50, E50 (acétate de vitamine E adsorbé sur silice),
- Nanocapsules sèches C24 de l'exemple 1.

Après l'administration par gavage des produits vitamine E mis dans une gélule, des prélèvements de sang ont été fait à 0, 6, 12, 24, 48 et 96 h post gavage. Après centrifugation, le plasma est décanté et le tocophérol libre est dosé par HPLC.

### Résultats

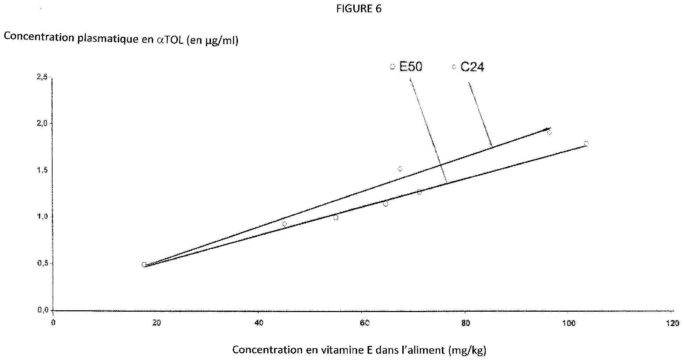

Les résultats sont reportés dans la figure 6.

Le gavage avec la formulation C24 a conduit à des concentrations plasmatiques en αTOL significativement plus élevées que le gavage avec le produit E50.

L'aliment gavage avec la formulation C24 a conduit à des concentrations hépatiques en αTOL significativement plus élevées (+24%) que le régime avec la formulation E50 sur la base de la comparaison des pentes de droite de l'effet dose-réponse.

## Revendications

1. Suspension colloïdale de nanocapsules, lesdites nanocapsules comprenant
une fraction huileuse consistant en un principe actif liposoluble et un tensioactif ionique,
un tensioactif non ionique,
et un polymère hydrophile, cationique ou anionique, entourant ladite fraction huileuse,
lesdites nanocapsules étant **caractérisées en ce que**
la charge du tensioactif ionique et celle du polymère hydrophile sont opposées, ledit tensioactif ionique étant choisi parmi les phosphatidylcholines et le bromure d'hexadécyl triméthylammonium,
le tensioactif non ionique est choisi parmi les copolymères à blocs polyoxyéthylène-polyoxypropylène, les mélange de copolymères à blocs polyoxyéthylène (EO)-polyoxypropylène (PO), le Tween 80, les esters d'acides gras et de saccharose, et tout mélange de ceux-ci, et
ledit polymère est choisi parmi le chitosane, l'alginate, la pectine, l'amidon, la cellulose, la caséine et leurs combinaisons.

2. Suspension colloïdale selon la revendication 1, **caractérisée en ce que** ledit principe actif est choisi parmi la vitamine A, la vitamine D, la vitamine E, la vitamine K et leurs dérivés ou leurs métabolites, les huiles essentielles, les acides gras, les huiles grasses et tout mélange de ceux-ci.

3. Suspension colloïdale selon la revendication 2, **caractérisée en ce que** ledit principe actif est choisi parmi les huiles essentielles de thym, d'origan, de romarin, d'ail, de camélia, de moutarde, de gingembre, de curcuma, de raisin, d'agrumes, de sainfoin, de yucca, d'armoise, de cannelle, de menthe, de clou de girofle, de baies, de cumin et d'Echinacea.

4. Suspension colloïdale selon l'une quelconque des revendications précédentes, **caractérisée en ce que** ledit tensioactif ionique présente un poids moléculaire d'au plus 1500 g/mol, de préférence d'au plus 1000 g/mol.

5. Suspension colloïdale selon l'une quelconque des revendications précédentes, **caractérisée en ce que** le tensioactif ionique est choisi parmi la lécithine d'œuf et la lécithine de soja.

6. Suspension colloïdale selon l'une quelconque des revendications 1 à 5, **caractérisé en ce que** le ou les copolymères à blocs polyoxyéthylène-polyoxypropylène sont choisis parmi les copolymères de formule EOₓ-PO_{y}-EOₓ dans laquelle x varie de 75 à 85 et y varie de 25 à 35, les copolymères de formule EOₓ-PO_{y}-EOₓ dans laquelle x varie de 55 à 65 et y varie de 35 à 45 et les copolymères de formule EOₓ-PO_{y}-EOₓ dans laquelle x varie de 112 à 123 et y varie de 40 à 50.

7. Suspension colloïdale selon l'une quelconque des revendications 1 à 5, **caractérisée en ce que** les esters d'acides gras et de saccharose sont choisis parmi les stéarates et les palmitates.

8. Suspension colloïdale selon l'une quelconque des revendications précédentes, **caractérisée en ce que** la teneur en dit principe actif est d'au moins 25% en masse par rapport à la masse sèche des nanocapsules, de préférence d'au moins 50%.

9. Nanocapsules comprenant
une fraction huileuse consistant en un principe actif liposoluble et un tensioactif ionique,
un tensioactif non ionique,
et un polymère hydrophile, cationique ou anionique, entourant ladite fraction huileuse,
lesdites nanocapsules étant **caractérisées en ce que**
la charge du tensioactif ionique et celle du polymère hydrophile sont opposées, ledit tensioactif ionique étant choisi parmi les phosphatidylcholines et le bromure d'hexadécyl triméthylammonium,
le tensioactif non ionique est choisi parmi les copolymères à blocs polyoxyéthylène-polyoxypropylène, les mélange de copolymères à blocs polyoxyéthylène (EO)-polyoxypropylène (PO), le Tween 80, les esters d'acides gras et de saccharose, et tout mélange de ceux-ci, et
ledit polymère est choisi parmi le chitosane, l'alginate, la pectine, l'amidon, la cellulose, la caséine et leurs combinaisons,
lesdites nanocapsules étant susceptibles d'être obtenues par séchage d'une suspension colloïdale selon l'une quelconque des revendications précédentes.

10. Nanocapsules selon la revendication 9, adsorbées sur du lactose.

11. Microparticules comprenant des nanocapsules selon la revendication 10.

12. Utilisation des nanocapsules selon la revendication 9 ou 10 ou des microparticules selon la revendication 11, en nutrition animale, notamment pour les monogastriques, dans lesquelles le principe actif est choisi parmi la vitamine A, la vitamine D, la vitamine E, la vitamine K et leurs dérivés ou leurs métabolites, les huiles essentielles, les acides gras, les huiles grasses et tout mélange de ceux-ci.

## Patentansprüche

1. Kolloidale Suspension von Nanokapseln, wobei die Nanokapseln
eine ölige Fraktion bestehend aus einem fettlöslichen Wirkstoff und einem ionischen Tensid,
ein nichtionisches Tensid,
und ein hydrophiles, kationisches oder anionisches Polymer, das die ölige Fraktion umgibt, umfassen,
wobei die Nanokapseln **dadurch gekennzeichnet sind, dass**
die Ladung des ionischen Tensids und die des hydrophilen Polymers entgegengesetzt sind, wobei das ionische Tensid aus Phosphatidylcholinen und Hexadecyltrimethylammoniumbromid ausgewählt ist,
das nichtionische Tensid aus Polyoxyethylen-Polyoxypropylen-Blockcopolymeren, Mischungen von Polyoxyethylen(EO)-Polyoxypropylen(PO)-Blockcopolymeren, Tween 80, Fettsäure- und Saccharoseestern und jeglichen Mischungen davon ausgewählt ist, und
das Polymer aus Chitosan, Alginat, Pektin, Stärke, Cellulose, Kasein und Kombinationen davon ausgewählt ist.

2. Kolloidale Suspension nach Anspruch 1, **dadurch gekennzeichnet, dass** der Wirkstoff aus Vitamin A, Vitamin D, Vitamin E, Vitamin K und deren Derivaten oder Metaboliten, ätherischen Ölen, Fettsäuren, fetten Ölen und Mischungen davon ausgewählt ist.

3. Kolloidale Suspension nach Anspruch 2, **dadurch gekennzeichnet, dass** der Wirkstoff aus den ätherischen Ölen von Thymian, Oregano, Rosmarin, Knoblauch, Kamelie, Senf, Ingwer, Kurkuma, Trauben, Zitrusfrüchten, Esparsette, Yucca, Beifuß, Zimt, Minze, Nelken, Beeren, Kreuzkümmel und Echinacea ausgewählt ist.

4. Kolloidale Suspension nach einem der vorhergehenden Ansprüche, **dadurch gekennzeichnet, dass** das ionische Tensid ein Molekulargewicht von höchstens 1500 g/mol, vorzugsweise von höchstens 1000 g/mol, aufweist.

5. Kolloidale Suspension nach einem der vorhergehenden Ansprüche, **dadurch gekennzeichnet, dass** das ionische Tensid aus Eilecithin und Sojalecithin ausgewählt ist.

6. Kolloidale Suspension nach einem der Ansprüche 1 bis 5, **dadurch gekennzeichnet, dass** das oder die Polyoxyethylen-Polyoxypropylen-Blockcopolymere aus den Copolymeren der Formel EOₓ-PO_{y}-EOₓ, wobei x von 75 bis 85 variiert und y von 25 bis 35 variiert, den Copolymeren der Formel EOₓ-PO_{y}-EOₓ, wobei x von 55 bis 65 variiert und y von 35 bis 45 variiert, und den Copolymeren der Formel EOₓ-PO_{y}-EOₓ, wobei x von 112 bis 123 variiert und y von 40 bis 50 variiert, ausgewählt ist/sind.

7. Kolloidale Suspension nach einem der Ansprüche 1 bis 5, **dadurch gekennzeichnet, dass** die Ester von Fettsäuren und Saccharose aus Stearaten und Palmitaten ausgewählt sind.

8. Kolloidale Suspension nach einem der vorhergehenden Ansprüche, **dadurch gekennzeichnet, dass** der Gehalt an dem Wirkstoff mindestens 25 Massenprozent bezogen auf die Trockenmasse der Nanokapseln beträgt, vorzugsweise mindestens 50 Massenprozent.

9. Nanokapseln, umfassend
eine ölige Fraktion bestehend aus einem fettlöslichen Wirkstoff und einem ionischen Tensid,
ein nichtionisches Tensid,
und ein hydrophiles, kationisches oder anionisches Polymer, das die ölige Fraktion umgibt,
wobei die Nanokapseln **dadurch gekennzeichnet sind, dass**
die Ladung des ionischen Tensids und die des hydrophilen Polymers entgegengesetzt sind, wobei das ionische Tensid aus Phosphatidylcholinen und Hexadecyltrimethylammoniumbromid ausgewählt ist,
das nichtionische Tensid aus Polyoxyethylen-Polyoxypropylen-Blockcopolymeren, Mischungen von Polyoxyethylen(EO)-Polyoxypropylen(PO)-Blockcopolymeren, Tween 80, Fettsäure- und Saccharoseestern und jeglichen Mischungen davon ausgewählt ist, und
das Polymer aus Chitosan, Alginat, Pektin, Stärke, Cellulose, Kasein und Kombinationen davon ausgewählt ist,
wobei die Nanokapseln durch Trocknen einer kolloidalen Suspension nach einem der vorhergehenden Ansprüche erhalten werden können.

10. Nanokapseln nach Anspruch 9, die an Lactose adsorbiert sind.

11. Mikropartikel, die Nanokapseln nach Anspruch 10 umfassen.

12. Verwendung der Nanokapseln nach Anspruch 9 oder 10 oder der Mikropartikel nach Anspruch 11 in der Tierernährung, insbesondere für Monogastriden, wobei der Wirkstoff aus Vitamin A, Vitamin D, Vitamin E, Vitamin K und deren Derivaten oder Metaboliten, ätherischen Ölen, Fettsäuren, fetten Ölen und Mischungen davon ausgewählt sind.

## Claims

1. A colloidal suspension of nanocapsules, said nanocapsules comprising
an oil fraction consisting of a fat-soluble active ingredient and an ionic surfactant,
a non-ionic surfactant,
and a hydrophilic, cationic or anionic polymer, surrounding said oil fraction,
said nanocapsules being **characterized in that**
the charge of the ionic surfactant and that of the hydrophilic polymer are opposite, said ionic surfactant being selected from phosphatidylcholines and hexadecyl trimethylammonium bromide,
the non-ionic surfactant is selected from polyoxyethylene-polyoxypropylene block copolymers, mixtures of polyoxyethylene (EO)-polyoxypropylene (PO) block copolymers, Tween 80, sucrose fatty acid esters, and any mixture thereof, and
said polymer is selected from chitosan, alginate, pectin, starch, cellulose, casein, and combinations thereof.

2. The colloidal suspension according to claim 1, **characterized in that** said active ingredient is selected from vitamin A, vitamin D, vitamin E, vitamin K and derivatives or metabolites thereof, essential oils, fatty acids, fatty oils, and any mixture thereof.

3. The colloidal suspension according to claim 2, **characterized in that** said active ingredient is selected from essential oils of thyme, oregano, rosemary, garlic, camellia, mustard, ginger, turmeric, grape, citrus, sainfoin, yucca, mugwort, cinnamon, mint, clove, berries, cumin, and Echinacea.

4. The colloidal suspension according to any one of the preceding claims, **characterized in that** said ionic surfactant has a molecular weight of at most 1500 g/mol, preferably at most 1000 g/mol.

5. The colloidal suspension according to any one of the preceding claims, **characterized in that** the ionic surfactant is selected from egg lecithin and soy lecithin.

6. The colloidal suspension according to any one of claims 1 to 5, **characterized in that** the polyoxyethylene-polyoxypropylene block copolymer(s) is/are selected from copolymers of formula EOₓ-PO_{y}-EOₓ in which x varies from 75 to 85 and y varies from 25 to 35, copolymers of formula EOₓ-PO_{y}-EOₓ in which x varies from 55 to 65 and y varies from 35 to 45 and copolymers of formula EOₓ-PO_{y}-EOₓ in which x varies from 112 to 123 and y varies from 40 to 50.

7. The colloidal suspension according to any one of claims 1 to 5, **characterized in that** the sucrose fatty acid esters are selected from stearates and palmitates.

8. The colloidal suspension according to any one of the preceding claims, **characterized in that** the content of said active ingredient is at least 25% by mass relative to the dry mass of the nanocapsules, preferably at least 50%.

9. Nanocapsules comprising
an oil fraction consisting of a fat-soluble active ingredient and an ionic surfactant,
a non-ionic surfactant,
and a hydrophilic, cationic or anionic polymer, surrounding said oil fraction,
said nanocapsules being **characterized in that**
the charge of the ionic surfactant and that of the hydrophilic polymer are opposite, said ionic surfactant being selected from phosphatidylcholines and hexadecyl trimethylammonium bromide,
the non-ionic surfactant is selected from polyoxyethylene-polyoxypropylene block copolymers, mixtures of polyoxyethylene (EO)-polyoxypropylene (PO) block copolymers, Tween 80, sucrose fatty acid esters, and any mixture thereof, and
said polymer is selected from chitosan, alginate, pectin, starch, cellulose, casein and combinations thereof,
said nanocapsules being likely to be obtained by drying a colloidal suspension according to any one of the preceding claims.

10. The nanocapsules according to claim 9, adsorbed on lactose.

11. Microparticles comprising nanocapsules according to claim 10.

12. A use of the nanocapsules according to claim 9 or 10 or of the microparticles according to claim 11, in animal nutrition, in particular for monogastric animals, in which the active ingredient is selected from vitamin A, vitamin D, vitamin E, vitamin K and derivatives or metabolites thereof, essential oils, fatty acids, fatty oils, and any mixture thereof.
